(19) Europäisches Patentamt
European Patent Office
Office européen des brevets

(11) Numéro de publication: **0 462 004 B1**

(12) # FASCICULE DE BREVET EUROPEEN

(45) Date de publication du fascicule du brevet: **06.09.95**

(51) Int. Cl.$^{6}$: **A61K 31/165**

(21) Numéro de dépôt: **91401563.1**

(22) Date de dépôt: **12.06.91**

(54) **Nouvelle utilisation du modafinil.**

(30) Priorité: **14.06.90 FR 9007442**

(43) Date de publication de la demande:
**18.12.91 Bulletin 91/51**

(45) Mention de la délivrance du brevet:
**06.09.95 Bulletin 95/36**

(84) Etats contractants désignés:
**AT BE CH DE DK FR GB IT LI LU NL SE**

(56) Documents cités:
**FR-A- 2 385 693**

**DRUGS FUTURE, vol. 15, no. 2, février 1990, pages 130-132; "Modafinil"**

(73) Titulaire: **LABORATOIRE L. LAFON**
**19 Avenue du Professeur Cadiot**
**F-94701 Maisons Alfort (FR)**

(72) Inventeur: **Lafon, Louis**
**5,rue de l'Alboni**
**F - 7500 Paris (FR)**

(74) Mandataire: **Le Guen, Gérard et al**
**CABINET LAVOIX**
**2, place d'Estienne d'Orves**
**F-75441 Paris Cédex 09 (FR)**

**EP 0 462 004 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne une nouvelle utilisation du modafinil.

Le modafinil ou benzhydrylsulfonyl acétamide est un composé de formule

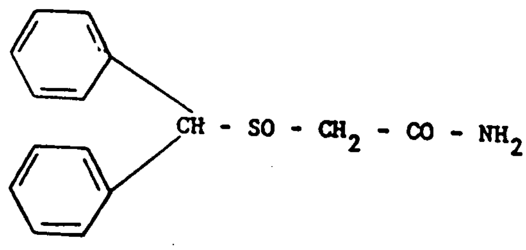

Ce composé et son application thérapeutique comme stimulant du système nerveux central ont été décrits dans FR-A-2 385 693.

On a maintenant découvert que le modafinil possède un effet neuroprotecteur qui peut être utilisé en thérapeutique, notamment dans le traitement de la maladie de Parkinson et les autres maladies dégénératives du système nerveux central.

La présente invention a en conséquence pour objet l'utilisation du modafinil pour la fabrication d'un médicament ayant un effet neuroprotecteur.

Le médicament neuroprotecteur contenant le modafinil peut être présenté notamment sous une forme convenant pour l'administration par voie orale. Généralement les doses administrées peuvent être de 50 à 1000 mg chez l'homme.

On donnera ci-après des résultats d'essais pharmacologiques mettant en évidence les effets neuroprotecteurs du modafinil.

Pour certains de ces essais, on a utilisé des souris auxquelles on a administré de la MPTP (1-méthyl-4-phényl-1,2,3,6-tétrahydropyridine). La MPTP déclenche chez la souris des symptômes parkinsoniens par destruction des voies nigrostriatales, suivie d'une déplétion en dopamine. La MPTP permet d'obtenir un modèle considéré comme fiable de la maladie de Parkinson.

1 - Effet du modafinil sur la disparition induite par la MPTP de la fixation du $^{3}$H-mazindol dans les membranes néostriatales chez la souris noire adulte.

On utilise la méthode décrite par Javitch et. al (Mol Pharmacol 26, p. 35, 1984).

On injecte la MPTP (1-méthyl-4-phényl-1,2,-3,6-tétrahydropyridine) par voie sous-cutanée à la dose de 40 mg/kg à des souris mâles adultes de souche C 57 bl/6. 15 minutes après on injecte le modafinil en suspension dans de la carboxyméthylcellulose sodique (0,4%) par voie intrapéritonéale et on répète l'injection 1 fois par jour pendant 14 jours.

On utilise le $^{3}$H-mazindol (15 Ci/mmol) un radioligand fixant la dopamine à une concentration de 60 nM. Le mazindole 10 $\mu$M a été utilisé pour définir les fixations spécifiques.

Le tableau I donne les résultats obtenus

| Dose Modafinil mg/kg | Fixation de $^3$H-Mazindol (néostriatum) dpm/mg prot* |
|---|---|
| 0 | 28810 ± 215 |
| 10 | 36820 ± 216 |
| 30 | 40130 ± 126 |
| 100 | 66460 ± 206 |

* dpm/mg prot : désintégration par minute/mg de protéine.

Ces résultats mettent en évidence un effet protecteur fonction de la dose du modafinil.

2 - Effet du modafinil sur la dégénération induite par la MPTP de terminaisons nerveuses DA striatales chez la souris noire.

On utilise la méthode décrite par Agnati et al (Neuroscience 26, 461, 1988).

On injecte la MPTP par voie intrapéritonale à la dose de 40 mg/kg. 15 minutes après on injecte le modafinil en suspension dans de la carboxyméthyl cellulose sodique à 0,4% par voie intraperitonéale et répète l'injection chaque jour. Les animaux sont sacrifiés 14 jours après l'injection de MPTP. On mesure l'immunoréactivité (IR) de la tyrosine hydroxylase par analyse d'image dans des échantillons de neostriatum médian.

Le tableau II donne les résultats obtenus.

| Dose modafinil mg/kg | Valeur d'immunoréactivité |
|---|---|
| 0 | 151 ± 17,8 |
| 10 | 364 ± 63,9 |
| 30 | 426 ± 99,2 |
| 100 | 520 ± 121 |

Ces résulats mettent en évidence que le modafinil exerce un effet protecteur contre la neuroxicité du MPTP et que cet effet est fonction de la dose.

Avec le même modèle expérimental on a également mesuré le stock de dopamine contenue dans les neurones de la substantia nigra.

Le tableau III donne les résultats obtenus:

| Traitement | Dopamine mg/g tissu |
|---|---|
| MPTP | 144 ± 16 |
| MPTP + modafinil 10 mg/kg | 309 ± 45 |
| MPTP + modafinil 100 mg/kg | 325 ± 26 |

Le modafinil s'oppose à la déplétion du taux de dopamine induite par le MPTP et cela d'une façon dépendant de la dose.

3 - Effet du modafinil sur la dégénérescence des neurones DA nigrostriataux à la suite d'une hémitransection partielle chez le rat mâle.

Le traitement par modafinil est débuté 15 minutes après l'hémitransection partielle (selon la méthode d'Agnati et al., Acta Physiol. Scand. 119, 347), à la dose de 30 $mg.kg^{-1}$ pendant une période de 15 jours consécutifs. Cette lésion chirurgicale entraîne une diminution du stock de dopamine avec une neurodégénérescence partielle des terminaisons nerveuses dopaminergiques dans la zone limbique subcorticale (nucleus accumbens et tuberculum olfactorium), une déplétion totale du stock de dopamine dans le néostriatum et des modifications dans le contenu en sérotonine dans la substantia nigra. On a observé une activité pharmacologique du modafinil portant sur la zone non-lésée par la section chirurgicale (substantia nigra) et à la fois sur les zones non lésées et lésées de la région limbique subcorticale.

## Revendications

**1.** Utilisation du modafinil pour la fabrication d'un médicament destiné au traitement des maladies dégénératives du système nerveux central.

**2.** Utilisation selon la revendication 1 pour la fabrication d'un médicament destiné au traitement de la maladie de Parkinson.

## Claims

**1.** Use of modafinil for the production of a medicament intended for treating degenerative diseases of the central nervous system.

**2.** Use according to claim 1 for the production of a medicament intended for the treatment of Parkinson's disease.

## Patentansprüche

**1.** Verwendung von Modafinil für die Herstellung eines Arzneimittels zur Behandlung von degenerativen Erkrankungen des Zentralnervensystems.

**2.** Verwendung nach Anspruch 1 zur Herstellung eines Arzneimittels zur Behandlung der Parkinsonschen Krankheit.